# EUROPÄISCHE PATENTSCHRIFT

(11) **EP 0 496 373 B1**
(45) Veröffentlichungstag und Bekanntmachung des Hinweises auf die Patenterteilung: **06.05.1998**
(21) Anmeldenummer: 92101000.5
(22) Anmeldetag: 22.01.1992
(51) Int. Cl.: C12P 21/02, C07C 237/12, C07K 1/06

(54) **Verfahren zur enzymatischen Herstellung geschützter und ungeschützter Di- und Oligopeptide in wässrigen Lösungen**
Process for preparing enzymatically protected and unprotected amino acids, di- and oligopeptides in aqueous solution
Procédé pour la préparation enzymatique d'acides aminés, di- et oligopeptides protégés et non-protégés dans des solutions aqueuses

(30) Priorität: 23.01.1991 DE 4101895
(43) Veröffentlichungstag der Anmeldung: 29.07.1992
(73) Patentinhaber: FORSCHUNGSZENTRUM JÜLICH GMBH, 52425 Jülich (DE); Degussa Aktiengesellschaft, 60311 Frankfurt (DE)
(72) Erfinder: Fischer, Andreas, W-5300 Bonn 1 (DE); Schwarz, Alexander c/o Dept. of Biophysics, 76100 Rehovot (IL); Wandrey, Christian, Prof., W-5170 Jülich (DE); Knaup, Günter, Dr., W-6454 Bruchköbel (DE); Bommarius, Andreas, Dr., W-6000 Frankfurt 70 (DE); Drauz, Karl-Heinz, Dr., W-6364 Freigericht (DE)

(56) Entgegenhaltungen:
- CH-A- 618 842
- COLLECTION OF CZECHOSLOVAK CHEMICAL COMMUNICATIONS. Bd. 53, Nr. 11, 1. November 1988, PRAGUE CS Seiten 2884 - 2889; V SCHELLENBERGER ET AL.: 'specific water- soluble substrates for chymotrypsin: attempts for compensating diminished p1-s1 interactions'
- CHEMICAL ABSTRACTS, vol. 103, no. 15, 14. Oktober 1985, Columbus, Ohio, US; abstract no. 121717T, H D JAKUBKE ET AL.: 'peptides' Seite 587 ;Spalte R ;
- CHEMICAL ABSTRACTS, vol. 114, no. 7, 18. Februar 1991, Columbus, Ohio, US; abstract no. 58418F, M YU GOLOLOBOV ET AL: 'increased nucleophile reactivity of amino acid beta-naphthylamides in alpha-chymotrypsin-catalyzed peptide synthesis' Seite 336 ;Spalte R ;

## Beschreibung

Die Erfindung bezieht sich auf ein Verfahren zur Herstellung von geschützten bzw. ungeschützten Di- oder Oligopeptiden durch enzymatische Umsetzung einer N-terminal geschützten C-terminal veresterten Aminosäure bzw. eines solchen Di-oder Oligopeptids (A) als Elektrophil mit einer Aminosäure oder einem Di- oder Oligopeptid oder einem entsprechenden C-terminalen Ester oder Amid (B) als Nucleophil in Gegenwart von Hydrolase (E.C. 3.4...) in wäßriger Lösung,

Synthetische kurzkettige Peptide finden in der Pharmakologie und in der parenteralen Ernährung zunehmend Verwendung. Als Beispiel für ein pharmakologisch wirksames Dipeptid sei Kyotorphin (L-Tyr-L-Arg) genannt, das die Freisetzung von Enkephalinen fördert, endogenen Substanzen also, die im Gehirn eine analgetische und beruhigende Wirkung besitzen (Hughes,1975).

Der chemischen und optischen Reinheit der für eine solche Anwendung vorgesehenen Peptide kommt somit eine hohe Bedeutung zu. Daher werden immer öfter enzymatische Peptidsynthesen, die in aller Regel regio- und stereospezifisch verlaufen, bevorzugt eingesetzt. Meist werden dabei N-geschützte Aminosäurealkylester in Gegenwart einer Hydrolase mit einem Aminosäure- oder Di- bzw. Oligopeptidderivat mit freier Aminogruppe in einer kinetisch kontrollierten Reaktion umgesetzt. Bei Durchführung der Reaktionen in Gegenwart von Wasser tritt die thermodynamisch begünstigte Hydrolyse der Alkylester als Konkurrenzreaktion auf. Nachteilig an diesem Verfahren ist die Tatsache, daß Enzyme nur in Gegenwart von Wasser aktiv und stabil sind und die zum Einsatz kommenden Substrate im allgemeinen nur eine geringe Wasserlöslichkeit aufweisen. Bei Zusätzen von organischen Lösungsmitteln als Löslichkeitsvermittler ist im allgemeinen mit einer zumindest partiellen Desaktivierung des Enzyms und einer Abnahme der Stabilität zu rechnen. Daher wurde bisher meist nur mit Substratkonzentrationen von maximal 100 mM gearbeitet. Zur Selektivitäts- und daher Ausbeutesteigerung werden bisher weiterhin die Nucleophilkomponenten in großen Überschüssen (2-20fach) eingesetzt. Im Vergleich zu den chemischen Synthesen kurzkettiger Peptide in flüssiger Phase, wobei Substrat und Nucleophil praktisch stöchiometrisch und in hohen Konzentrationen eingesetzt werden, war ein solches Arbeiten bei enzymatischen Synthesen bisher nicht durchführbar.

Die Patentschrift DD 218 904 A1 beschreibt ein Verfahren zur Herstellung von Peptiden bei dem auf eine Löslichkeitsverbesserung durch eine entsprechend N-terminale Schutzgruppe beim Elektrophil hingewiesen wird. Jedoch erfordert die Umsetzung meist ein unter Zusatz von organischem Lösungsmittel verändertes Reaktionsmilieu.

Ziel der Erfindung war daher, ein Verfahren zu entwickeln, das es erlaubt, die Enzymreaktionen mit vergleichbar hohen Konzentrationen auch im wäßrigen Milieu durchzuführen und vorzugsweise auch die dafür benötigten Überschüsse an Nucleophilkomponente drastisch zu reduzieren.

Dieses Ziel wird erfindungsgemäß dadurch erreicht, daß als Elektrophil (A) eine Aminosäure, ein Dipeptid oder ein Oligopeptid in einer Konzentration von ≥ 100 mM eingesetzt wird, welche bzw. welches eine die Wasserlöslichkeit um einen Faktor > 5 gegenüber den ungeschützten Elektrophil (A) erhöhende, N-terminale Schutzgruppe und/oder eine entsprechende C-terminale Ester- oder Amidgruppe aufweist. Außerdem wird als Nucleophil (B) eine Aminosäure oder ein Di- oder Oligopeptide eingesetzt, welche bzw. welches ggt. als C-terminale Ester- oder C-terminales Amid vorliegt. Gegebenenfalls, wird das von der Reaktionsmischung abgetrennte Reaktionsprodukt von Schutzgruppen befreit und isoliert.

Insbesondere wird eine Verbindung der allgemeinen Formel I

X-E-R¹ (I),

in der E unter Einbezug von X und R¹ für eine N-terminal mit X und C-terminal mit R¹ substituierte Aminosäure bzw. ein solches Di- oder Oligopeptid steht, wobei
- R¹: eine veresternde Alkylgruppe mit 1 - 4 C-Atomen bedeutet, die ggf. eine Arylgruppe als Substituenten aufweist und
- X: eine bei den zur Umsetzung angewandten pH-Werten die Wasserlöslichkeit gegenüber Verbindungen mit X = H um einen Faktor > 5 erhöhende Gruppe ist,
als Elektrophil
mit einer Verbindung der allgemeinen Formel II

H₂N-Q-R² (II)

als Nucleophil, in der Q unter Einbezug von H₂N und R² für eine C-terminal eine R²-Gruppe tragende Aminosäure oder ein solches Di- oder Oligopeptid steht, wobei
- R²: ein freies Säureende, eine ggf. eine Arylgruppe als Substituenten aufweisende C₁- bis C₄-Alkylveresterung oder eine Amidierung durch -NR³R⁴ symbolisiert, deren Reste R³ und R⁴ jeweils unabhängig voneinander Wasserstoff, einen C₁- bis C₄-Alkylrest, einen solchen Arylalkylrest oder einen Arylrest bedeuten,
in wäßriger Lösung in Gegenwart einer Hydrolase umgesetzt wird, wobei das Elektrophil (I) in ≥ 100 mM Konzentration eingesetzt wird
und wobei ggf. R¹ und/oder R²,abweichend von der vorstehenden Definition derselben, die Funktion der Löslishkeitsvermittlung im wässrigen Milieu an Stelle von oder zusätzlich zu der Gruppe X, insb. durch kationische oder anionische Struktur übernehmen bzw. mitübernehmen und X dann ggf. eine beliebige N-terminale Schutzgruppe ist
und daß ggf. das von der Reaktionsmischung abgetrennte Reaktionsprodukt von Schutzgruppen befreit wird.

Es wurde festgestellt, daß die Wasserlöslichkeit von Aminosäure- bzw. Peptidalkylestern (des Elektrophil) durch bestimmte N-terminale insb. polare bzw. ladungstragende Schutzgruppen nicht nur so extrem gesteigert werden kann (z.B. für Tyrosinethylester auf das 5- bis 100-fache), daß man mit hohen Substratkonzentrationen > 50 mM, vorzugsweise > 100 mM, und meist bis 1 M, aber auch höher (z.B. bis zu 4 M) in homogener Phase arbeiten kann, sondern daß diese hohen Substratkonzentrationen darüber hinaus überraschenderweise zu extrem erhöhten Enzymaktivitäten führen. Als N-terminale Schutzgruppen eignen sich polare Gruppen, die vorzugsweise ladungstragend sind. Unter polar versteht man solche funktionellen Gruppen, deren Elektronenverteilung der Gruppe ein solches elektrisches Diplomoment erteilen, das geeignet ist, obige Bedingung zu erfüllen. Solche Gruppen bedingen eine Affinität zu Wasser und steigern hierdurch den hydrophilen Charakter der Substanz, so daß in rein wäßrigen Lösungen gearbeitet werden kann.

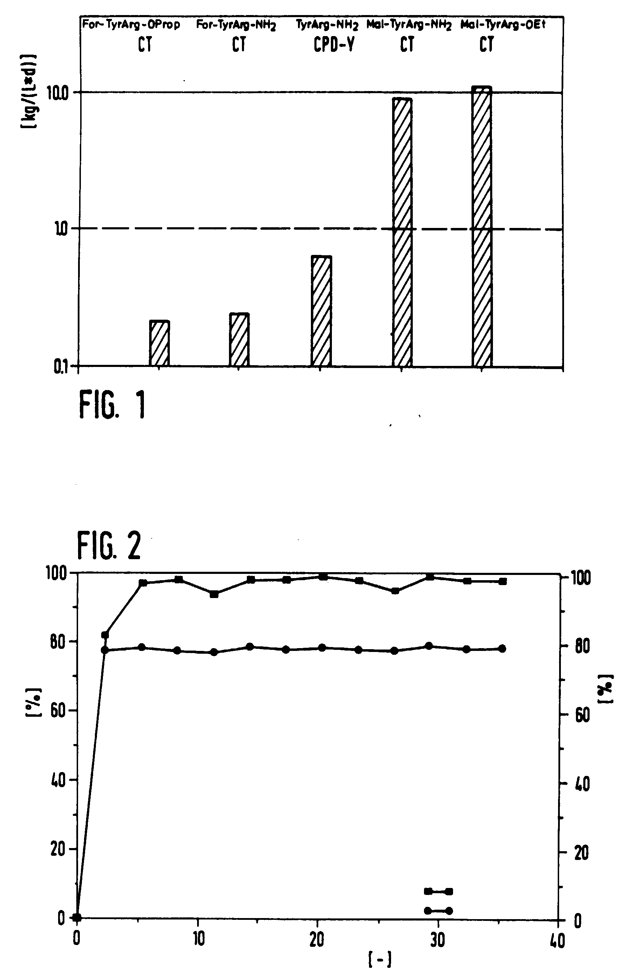

Durch das erfindungsgemäße Verfahren können damit äußerst hohe Raum-Zeit-Ausbeuten erreicht werden. Diese liegen im Vergleich zum derzeitigen Stand der Technik z.B. für die Synthese von geschütztem Tyr-Arg um den Faktor 10 höher (Fig. 1). Als ein weiterer Effekt der drastisch erhöhten Substratkonzentrationen wurde überraschend festgestellt, daß der Anteil an Nucleophilkomponente bis auf äquimolare Mengen reduziert werden kann, ohne die Selektivität im Vergleich zur Hydrolysereaktion nennenswert zu verringern. Vorzugsweise wird die Verbindung der Formel II zur Verbindung der Formel I in einem Molverhältnis < 1,5 und insbesondere von 0,8 bis 1,2, besonders bevorzugt etwa 1,0, eingesetzt.

Für die Synthese werden insbesondere von natürlichen α-Aminosäuren abgeleitete Reaktionspartner verwendet. Bei der Wahl der ladungstragenden Schutzgruppe spielt die Art der Ladung (positiv/negativ) keine Rolle. So können N-terminal carboxysubstituierte Acylgruppen wie Phthalyl oder Maleyl, sowie Derivate bzw. Homologe derselben zum Einsatz kommen. Sie sind chemisch leicht einführbar und nach erfolgter Peptidsynthese unter milden Bedingungen, z.B. säurekatalytisch, wieder abspaltbar.

Zwar sind vor einiger Zeit Untersuchungen zur Substratspezifität von α-Chymotrypsin durchgeführt worden, bei denen auch N-Maleylaminosäureester für die Dipeptidsynthese verwendet wurden (V. Schellenberger, U. Schellenberger, H.-D. Jakubke, Coll. Czech. Chem. Commun. 53 (1988), 2884), wobei jedoch durchweg nur mit den bislang in Wasser üblichen niederen Substratkonzentrationen von 0,01 bis maximal 95 mM gearbeitet und die enorme positive Wirkung der durch Einführung der geladenen Schutzgruppe möglichen Steigerung der Substratkonzentration nicht erkannt wurde.

Als elektrophiler Reaktionspartner mit positiv geladener Schutzgruppe können z.B. die bisher nicht bekannten N-Betainyl-aminosäure- bzw. -peptidester der allgemeinen Formel IV 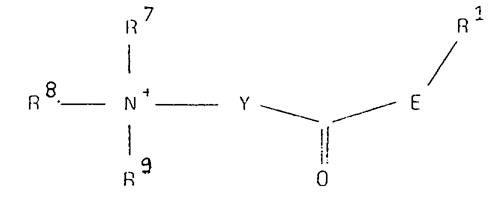 wobei 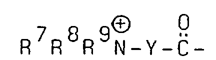 gleich X ist,
- E, X und R¹: die bereits angegebene Bedeutung haben,
- Y: eine Alkylengruppe mit 1 - 4 C-Atomen, Arylen-oder Aralkylengruppe darstellt und
- R⁷, R⁸ und R⁹: jeweils unabhängig voneinander für einen Alkylrest mit 1 - 4 C-Atomen oder einen Arylrest stehen, die ggf. substituiert sein können,
eingesetzt werden. Diese Derivate sind einfach durch Aminolyse der korrespondierenden Halogenacylverbindungen mit Trialkylaminen herstellbar. Die Betainylverbindungen mit Y = -CH₂- sind z.B. durch Umsetzung der literaturbekannten N-Chloracetyl-aminosäurealkylester mit Trialkylaminen zugänglich.

Als Enzyme können alle für Peptidsynthesen geeigneten Hydrolasen (E.C. 3.4..), wie z.B. α -Chymotrypsin, Subtilisin; Carboxypeptidase-W, -C und -Y; Trypsin; Papain, Ficin, Bromelain verwendet werden. Wenn als Elektrophil ein Derivat einer aromatischen Aminosäure, wie Tyrosin, Phenylalanin oder Tryptophan, eingesetzt wird, hat sich α-Chymotrypsin besonders bewährt. Für die Umsetzung der freien Säure gemäß Formel II, die im Hinblick auf die Aufarbeitung der Produktlösung favorisiert wird, ist Papain unter den bekannten Bedingungen speziell geeignet.

Die Substratkonzentration kann über 1 M hinaus bis zu 4 - 5 M gesteigert werden, es wurde jedoch vornehmlich mit Konzentrationen im Bereich von 0,5 bis 1 M gearbeitet.

Das erfindungsgemäße Verfahren erlaubt es im allgemeinen, bei einem Elektrophil/Hydrolase-Verhältnis im Bereich von 10⁵ bis 10⁷ (M/M) zu arbeiten, wobei die Reaktionszeiten im Vergleich zu herkömmlichen Verfahren, bei denen die Verhältnisse in der Regel bei 10² bis 10⁴ liegen, nicht wesentlich länger sind. Im Falle von Papain als Hydrolase ist ein Verhältnis ≥ 10³ (M/M) bevorzugt.

Das erfindungsgemäße Verfahren kann sowohl kontinuierlich als auch diskontinuierlich durchgeführt werden. Bei kontinuierlicher Fahrweise, z.B. in einem Enzym-Membran-Reaktor (EMR), sind mit dem erfindungsgemäßen Verfahren Umsätze von > 95 % bei Verweilzeiten von < 20 min erreichbar, insbesondere bei obigen Verhältnissen.

Für X ist insbesondere eine Gruppe der allgemeinen Formel III 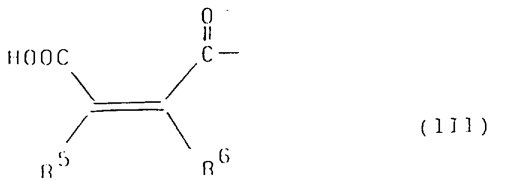 geeignet, wobei R⁵ und R⁶ jeweils unabhängig voneinander H, einen C₁ - C₄-Alkyl-, Aryl-, Heteroaryl-, Arylakyl-, Heteroarylalkylrest oder unter Einbeziehung der Doppelbindung zusammen einen ggf. substituierten aromatischen oder alicyclischen Ring bilden, der ggf. auch gesättigt sein kann.

Im Falle von R⁵ und R⁶ = H sollte das Elektrophil in einer Konzentration ≥ 100 mM vorliegen.

Überraschenderweise wurde weiter festgestellt, daß bei der Umsetzung einer Verbindung der allgemeinen Formel I, bei der E ein Tyrosin-, Phenylalanin- oder Tryptophanrest ist und X einen ggf. niederalkylierten (d.h. C₁ - C₄-alkylierten) Maleyl- oder einen ggf. substituierten Phthalylrest bedeutet,mit einem Argininalkylester als Nucleophil, das geschützte Dipeptidderivat in der Regel direkt aus der wäßrigen Reaktionslösung ausfällt und daher leicht abtrennbar ist. Ähnliche Vorteile können allgemein erzielt werden, wenn als löslichkeitsvermittelnde Gruppe im Elektrophil wesentlich R¹ wirkt, das durch die Umsetzung abgetrennt wird, so daß die resultierende geringere Löslichkeit des gewünschten Produkts zu seiner Ausfällung führt.

In Fällen, in denen die Wasserlöslichkeit des Nucleophils limitierend wirkt, kann in Fortführung des erfindungsgemäßen Verfahrens diese durch C-terminale Einführung einer die Wasserlöslichkeit erhöhenden, bei den zur Umsetzung angewandten pH-Werten insb. ladungstragenden Gruppe (R²), z.B. einer Cholinestergruppe, erhöht werden.
Analog besteht die Möglichkeit, die Erhöhung der Wasserlöslichkeit des Elektrophils statt über die Gruppe X oder zusätzlich durch eine C-terminale Gruppe R¹ zu erreichen, welche die Wasserlöslichkeit (insb. durch kationische oder anionische Struktur) erhöht.

Nachfolgend wird die Erfindung anhand von Beispielen näher erläutert:

### Beispiel 1: Synthese von N-Maleylaminosäureethylester

Die Darstellung der N-Maleylaminosäureethylester erfolgte nach einer Vorschrift von Atassi et al. (Methods of Enzymology 25 (1972), 546). Es werden die Ethylester von Tyrosin, Phenylalanin, Tryptophan, Alanin und Arginin umgesetzt.

Es werden je 200 mmol der Aminosäureesterhydrochloride in 500 mℓ H₂O gelöst und anschließend die Lösung mit NaOH auf pH 8,2 gestellt. Bei Raumtemperatur werden 300 mmol Maleinsäureanhydrid portionsweise zugegeben, wobei der pH mittels 5N NaOH auf pH 8,2 gehalten wird. Nach beendeter Zugabe wird die Reaktionslösung noch weitere 30 min bei Raumtemperatur und pH 8,2 gerührt. Die Umsatzkontrolle erfolgte mittels HPLC und zeigte in allen Fällen quantitativen Umsatz an. Die so erhaltene Lösung kann direkt zur enzymatischen Synthese umgesetzt werden. Ein festes, mit Natriumchlorid und Maleinsäure verunreinigtes Produkt kann durch Gefriertrocknung erhalten werden.

Löslichkeitsuntersuchungen in Wasser (Eigen-pH-Wert) brachten folgende Ergebnisse:

| | |
|---|---|
| Mal-Tyr-OEt: > 600 mM | Mal-Ala-OEt: > 1900 mM |
| Mal-Phe-OEt: > 700 mM | Mal-Arg-OEt: > 1000 mM |
| Mal-Trp-OEt: > 700 mM | |

### Beispiel 2: Synthese von N-Citraconyl-aminosäureethylester

Die Darstellung erfolgte analog Beispiel 1, mit der Maßgabe, daß anstelle von Maleinsäureanhydrid Citraconsäureanhydrid eingesetzt wird.

Löslichkeitsuntersuchungen in Wasser (Eigen-pH-Wert) erbrachten folgende Ergebnisse:

| | |
|---|---|
| Cit-Tyr-OEt | > 1000 mM |
| Cit-Phe-OEt | > 1000 mM |
| Cit-Trp-OEt | > 900 mM |

### Beispiel 3: Synthese von N-Phthalylaminosäureester

Die Darstellung erfolgte analog Beispiel 1, mit der Maßgabe, daß anstelle von Maleinsäureanhydrid Phthalsäureanhydrid eingesetzt wird. Es werden die Ethlester von Tyrosin, Alanin und Arginin umgesetzt.

Löslichkeitsuntersuchungen in Wasser (Eigen-pH-Wert) erbrachten folgende Ergebnisse:

| | |
|---|---|
| Phthal-Tyr-OEt | > 800 mM |
| Phthal-Ala-OEt | > 1800 mM |
| Phthal-Arg-OEt: | > 800 mM |

### Beispiel 4: Synthese von N-Trimellit-ylaminosäureester (TMS-Ester)

Die Darstellung erfolgte analog Beispiel 1, mit der Maßgabe, daß anstelle von Maleinsäureanhydrid Trimellitsäureanhydrid eingesetzt wird. Es wird der Ethylester von Tyrosin umgesetzt. Es wird eine Wasserlöslichkeit von > 1000 mM festgestellt.

### Beispiel 5: Synthese von N-(Trimethylammoniumacetyl)- aminosäureethylester-hydrochloriden

In einem 500 ml 3-Hals-Kolben mit KPG-Rührer, Kühler, Thermometer und Gaseinleitungsrohr werden die entsprechenden Chloracetylverbindungen (ClAc-Tyr-OEt 50 mmol; ClAc-Phe-OEt 100 mmol; ClAc-Trp-OEt 100 mmol), gelöst in 250 ml THF, vorgelegt und Trimethylamin gasförmig eingeleitet. Die Reaktionslösung wird jeweils 6 Std. refluxiert, wobei die jeweiligen Betainaminosäureethylester-hydrochloride ausfallen. Nach beendeter Reaktion wird abfiltriert, gründlich mit THF gewaschen und über P₂O₅ getrocknet.

N-(Trimethylammonium-acetyl)-L-tyrosin-ethylesterhydrochlorid (C₁₆H₂₆ClN₂O₄):
Ausbeute: 35 % d.Th.

| | | | | |
|---|---|---|---|---|
| Ber.: | C 55,73 | H 7,31 | N 8,12 | Cl 10,29 |
| Gef.: | C 55,56 | H 7,31 | N 8,41 | Cl 10,42 |

- ¹H-NMR (CDCl₃):: 1,15 (t, 3 H), 2,70 - 3,00 (m, 2H), 3,17 (s, 9H), 4,08 (q, 2 H), 4,17 (d, 1H), 4,27 (d, 1H), 4,45 (m, 1H), 6,66 (d, 2H), 7,03 (d, 2H), 9,35 (br, s, 2H).

N-(Trimethylammonium-acetyl)-L-phenylalanin-ethylester hydrochlorid (C₁₆H₂₆ClN₂O₃):
Ausbeute: 58 % d.Th.

| | | | | |
|---|---|---|---|---|
| Ber.: | C 58,44 | H 7,66 | N 8,51 | Cl 10,79 |
| Gef.: | C 58,39 | H 7,89 | N 8,61 | Cl 10,89 |

- ¹H-NMR (CDCl₃):: 1,27 (t, 3 H), 3,00 - 3,35 (m, 2H), 3,32 (s, 9H), 4,18 (q, 2H), 4,51 (d, 1H), 4,69 (m, 1H), 4,94 (d, 1H) 7,12 - 730 (m, 3H), 7,42 (m, 2H), 9,90 (d, 1H).

N-Trimethylammonium-acetyl)-L-tryptophan-ethylester-hydrochlorid (C₁₈H₂₉ClN₃O₃):
Ausbeute: 100 % d.Th.

| | | | | |
|---|---|---|---|---|
| Ber.: | C 58,77 | H 7,12 | N 11,42 | Cl 9,65 |
| Gef.: | C 57,96 | H 7,56 | N 10,69 | Cl 9,42 |

- ¹H-NMR (CDCl₃):: 1,26 (t, 3 H), 3,05 (s, 9H), 3,20 - 3,45 (m, 2H),4,18 (q, 2H), 4,38 (d, 1H), 4,58 (d, 1H), 4,72 (m, 1H), 7,08 (m, 2H), 7,38 (m, 1H), 7,54 (m, 2H), 9,37 (d, 1H), 9,91 (s, 1H).

Löslichkeitsuntersuchungen erbrachten für alle drei Betainaminosäureethylester-hydrochloride eine Wasserlöslichkeit von > 4 M.

### Beispiel 6: Synthese von N-(Trimethylammoniumpropionyl, BetProp)-aminosäure-ethylester-hydrochloriden

Die Darstellung erfolgte analog Beispiel 5, mit der Maßgabe, daß anstelle der Chloracetyl- die entsprechende Chlorpropionylverbindung eingesetzt wird. Es wird der Ethylester von Tyrosin umgesetzt. Es wird eine Wasserlöslichkeit von > 1000 mM festgestellt.

### Beispiel 7: Synthese von N-(Trimethylammoniumbutyryl, BetBut)-aminosäure-ethylesterhydrochloriden

Die Darstellung erfolgte analog Beispiel 5, mit der Maßgabe, daß anstelle der Chloracetyl- die entsprechende Chlorbutyrylverbindung eingesetzt wird. Es wird wiederum der Ethylester von Tyrosin umgesetzt. Es wird eine Wasserlöslichkeit von > 2200 mM festgestellt.

### Beispiel 8: Bestimmung der Aktivitäten und Selektivitäten der enzymatischen Umsetzungen von N-Maleyl-, N-Citraconyl-, N-Phthalyl-, N-Trimellityl-, N-(Trimethylammoniumacetyl)-, N-(Trimethylammoniumpropionyl)- und N-(Trimethylammoniumbutyryl)-aminosäureethylestern mit Arginin, Argininethylester und Agininamid

### Enzymatische Dipeptidsynthese

Als Enzyme wurden α-Chymotrypsin (CT), Carboxypeptidase Y (CPD-Y) und Papain eingesetzt, deren pH- und Temperaturoptima für die Dipeptidsynthese bekannt sind (pH 9,5; T = 25^{o}C für CT; pH 9,0 T = 25^{o}C für CPD-Y und Papain).
Daher wurden alle Dipeptidsynthesen unter diesen Reaktionsbedingungen unter jeweiliger Verwendung von äquimolaren Substrat- und Nucleophilkonzentrationen durchgeführt.

Die Substrat- und Nucleaphilkonzentrationen betrugen bei Verwendung von Arg-NH₂ jeweils 500 bzw. 300 mM und bei Verwendung von Arg-OEt 600 bzw. 300 mM. Zusätzlich wurde die Umsetzung mit 300 mM freiem Arginin mittels Papain durchgeführt. Die Substrat/Enzym-Verhältnisse (M/N) betrugen bei ArgNH₂ als Nucleophil 1,1·10⁷ (CT) bzw. 3·10⁵ (CPD-Y), bei Arg-OEt 2,4·10⁷ (CT) und bei Arg-OH 5,3·10³ (Papain).

Die mit diesen Reaktionsparametern nach Standardmethoden ermittelten Anfangsaktivitäten und Selektivitäten sind in der nachfolgenden Tabelle aufgeführt.

| Dipeptid | | Enzym | Selektivität | Anfangsaktivität |
|---|---|---|---|---|
| 1 | Mal-Tyr-Arg-NH₂ | α-CT | 47 % | 1500 U/mg |
| 2 | Mal-Phe-Arg-NH₂ | α-CT | 18 % | 155 U/mg |
| 3 | Cit-Trp-Arg-NH₂ | α-CT | 22 % | 410 U/mg |
| 4 | Mal-Trp-Arg NH₂ | α-CT | 42 % | 1420 U/mg |
| 5 | Bet-Tyr-Arg-NH₂ | α-CT | 29 % | 800 U/mg |
| 6 | Bet-Phe-Arg-NH₂ | α-CT | 42 % | 2200 U/mg |
| 7 | Cit-Phe-Arg-NH₂ | α-CT | 41 % | 1100 U/mg |
| 8 | Bet-Trp-Arg-NH₂ | α-CT | 13 % | 195 U/mg |
| 9 | Mal-Tyr-Arg-NH₂ | α-CT | 80 % | 3750 U/mg |
| 10 | Cit-Tyr-Arg-NH₂ | α-CT | 78 % | 2700 U/mg |
| 11 | Bet-Tyr-Arg-NH₂ | CPD-Y | 44 % | 57 U/mg |
| 12 | Bet-Phe-Arg-NH₂ | CPD-Y | 52 % | 185 U/mg |
| 13 | Bet-Trp-Arg-NH₂ | CPD-Y | 29 % | 29 U/mg |
| 14 | Phthal-Tyr-Arg-NH₂ | α-CT | 90 % | 900 U/mg |
| 15 | Phthal-Tyr-Arg-OEt | α-CT | 70 % | 190 U/mg |
| 16 | TMS-Tyr-Arg-NH₂ | α-CT | 85 % | 18 U/mg |
| 17 | TMS-Tyr-Arg-OEt | α-CT | 70 % | 12 U/mg |
| 18 | BetProp-Tyr-Arg-NH₂ | α-CT | 50 % | 2200 U/mg |
| 19 | BetBut-Tyr-Arg-NH₂ | α-CT | 70 % | 2500 U/mg |
| 20 | Phthal-Ala-Arg | Papain | 63 % | |

Bei den Umsetzungen 14 bis 19 wurden die Reaktionen jeweils in 300 mM Kontrationen durchgeführt. Die Substrat/Enzym-Verhältnisse (M/M) betrugen dann bei Arg-NH₂ 6,3·10⁶ (CT) und bei Arg-OEt 8,3·10⁶ (CT).

### Beispiel 9: Enzymatische Synthese von Mal-Tyr-Lys-OEt

In einem 100-ml Becherglas wurde mit Mal-Tyr-OEt als Elektrophil und Lys-OEt · 2 HCl als Nukleophil mit CT als Katalysator Mal-Tyr-Lys-OEt hergestellt. Nach kurzer Zeit fiel das Produkt als weißer Niederschlag aus und wurde abfiltriert, mit Wasser gewaschen und bei 50°C im Vakuum getrocknet. Die Bedingungen und Ergebnisse sind in folgender Tabelle zusammengestellt:

| | |
|---|---|
| Elektrophil-Nukleophil-Verhältnis | 1:1 |
| Substratkonzentration | je 0,4 M |
| Substrat-Enzym-Verhältnis | 1,5·10⁶ |
| pH-Wert | 9,5 |
| Temperatur | 25°C |
| Reaktionszeit | 10 Minuten |
| Isolierausbeute | 81 % |
| Raum-Zeit-Ausbeute | 19,5 kg/l.d |
| spez. Enzymaktivität | 4042 U/mg |

Als Nukleophil kann erfindungsgemäß auch ein ungeschütztes Dipeptid eingesetzt werden:

### Beispiel 10: Herstellung von Mal-Tyr-Ala-Gln

Mit Hilfe von CT als Katalysator wurde mit Mal-Tyr-OEt als Elektrophil und dem Dipeptid Ala-Gln als Nukleophil das geschützte Tripeptid Mal-Tyr-Ala-Gln hergestellt. Die Reaktionszeit betrug 20 Minuten, nach sechs Minuten wurde im HPLC keine Abnahme von Ala-Gln mehr registriert. Das Produkt wurde über Anionenaustauscher M 600 (Lewatit) von Ala-Gln getrennt und durch ¹H-NMR (Lsgsm.: D₂O) identifiziert. Die Ausbeute betrug 71,5 %.

| | |
|---|---|
| Elektrophil-Nukleophil-Verhältnis | 1:1 |
| Substratkonzentration | je 0,43 M |
| Substrat-Enzym-Verhältnis | 2,9·10⁵ |
| pH-Wert | 9,5 |
| Temperatur | 25°C |

Als Nukleophil kann erfindungsgemäß auch eine nichtproteinogene Aminosäure eingesetzt werden:

### Beispiel 11: Herstellung von Mal-Tyr-Orn-OEt

Mal-Tyr-OEt als Elektrophil und Orn-OEt · 2 HCl als Nukleophil wurden mit α-CT als Katalysator umgesetzt. Nach 20 Minuten Reaktionszeit wurde die Lösung eingeengt, das Enzym durch Ultrafiltration über eine Membran mit MWCO 10.000 abgetrennt und der gelöste Anteil lyophilisiert. Sowie Mal-Tyr-Orn als auch das Orn-Delta-Lactam-Zyklisierungsprodukt wurden durch präparative MPLC isoliert und durch ¹H-NMR identifiziert. Die Ausbeute der beiden Produkte (ca. 1:1) betrug zusammen etwa 45-50 %.

| | |
|---|---|
| Elektrophil-Nukleophil-Verhältnis | 1:1 |
| Substratkonzentration | je 0,26 M |
| Substrat-Enzym-Verhältnis | 6,5·10⁵ |
| pH-Wert | 9,5 |
| Temperatur | 25°C |

Als Nukleophil kann erfindungsgemäß auch eine nichtbasische Aminosäure eingesetzt werden:

### Beispiel 12: Herstellung von H-Tyr-Cit-OEt

Mit dem Enzym α-CT und Mal-Tyr-OEt als Elektrophil sowie Gitrullinethylester (Cit-OEt) als Nukleophil wurde in einem 100ml Becherglas Tyr-Git-OEt hergestellt. Nach 30 Minuten Reaktionszeit wurde das Enzym über ein Ultrafilter (10.000 MWCO) abgetrennt und das Produkt neben den Edukten Mal-Tyr-OEt und Cit-OEt sowie H-Tyr-OEt in gleichen Teilen über LC-MS nachgewiesen.

| | |
|---|---|
| Elektrophil-Nukleophil-Verhältnis | 1:1 |
| Substratkonzentration | je 0,12 M |
| Substrat-Enzym-Verhältnis | 1,5·10⁵ |
| pH-Wert | 10,2 |
| Temperatur | 25°C |

Als Enzym kann erfindungsgemäß neben den Serinproteasen auch eine Thiolprotease (Papain) verwendet werden sowie ein geschütztes Dipeptid (Mal-Tyr-Lys-OEt) als Elektrophil:

### Beispiel 13: Herstellung von Mal-Tyr-Lys-Ala-OMe

Aus Mal-Tyr-Lys-OEt als Elektrophil und Ala-OMe als Nukleophil wurde mit Papain als Katalysator in wäßriger Suspension das Produkt hergestellt. Nach 75 Minuten Reaktionszeit war noch 49 % Mal-Tyr-Lys-OEt vorhanden. Das Filtrat der Suspension wurde durch ein 10.000 MWCO Membranmodul ultrafiltriert und das Produkt durch LC-MS nachgewiesen.

| | |
|---|---|
| Elektrophil-Nukleophil-Verhältnis | 1:1 |
| Substratkonzentration | je 0,13 M |
| Substrat-Enzym-Verhältnis | 1,9·10³ |
| pH-Wert | 9,6 |
| Temperatur | 25°C |

### Beispiel 14: Kontinuierliche enzymatische Synthese von Mal-Tyr-Arg-OEt

In einem 10 mℓ Enzym-Membran-Reaktor wurde kontinuierlich über 7 Stunden Mal-Tyr-Arg-OEt hergestellt. Die Betriebsbedingungen sind in der folgenden Tabelle zusammengestellt:

| | |
|---|---|
| Enzym | α-Chymotrypsin |
| Enzymkonzentration | 0,2 mg/mℓ |
| Mal-Tyr-OEt | 600 mM |
| Arg-OEt | 600 mM |
| pH-Wert | 9,5 |
| Temperatur | 25°C |
| Verweilzeit | 12 min |
| Volumenstrom | 50 ml/h |
| Reaktorvolumen | 10 ml |
| mittlerer Umsatz | ca. 98 % |
| mittlere Selektivität | ca. 80 % |
| Raum-Zeit-Ausbeute | 13,36 kg(l^{.}d) |
| Enzymverbrauch | 97 mg/kg Produkt |

Die bei diesem Versuch erzielbaren Umsätze und Selektivitäten in Abhängigkeit von den eingestellten Verweilzeiten sind in Abb. 2 dargestellt.

Aus der auslaufenden Produktlösung fiel im vorgekühlten Auffanggefäß das geschützte Dipeptid aus, und besaß nach Filtration und mehrmaligem Waschen mit Wasser eine Reinheit von > 97 %.
- ¹H-NMR (D₂O + DCl):: 1,08 (t, 3H), 1,42 (m, 2H), 1,57 (m, 1H), 1,72 (m, 2H), 2,89 (m, 2H), 3,02 (m, 2H), 4,01 (q, 2H), 4,21 (m, 1H), 4,46 (m, 1H), 6,18 (d, 1H), 6,34 (d, 1H), 6,69 (d, 1H), 7,01 (d, 1H).

### Beispiel 15:

In einem Batch-Ansatz (300 l) werden Mal-Tyr-OEt (0,25 M) und Arg-OEt (0,25 M) bei pH 9,5 und 25°C mit α-Chymotrypsin (0.025 mg/ml) umgesetzt. Nach 2 Stunden erhält man durch Abfiltrieren des Niederschlages und Nachwaschen mit Wasser 26,0 kg Mal-Tyr-Arg-OEt (75 % d. Th.) mit einer HPLC-Reinheit von > 97 %.

### Beispiel 16: Synthese von Benzyloxycarbonylaminosäurecholinesterjodid

Die Darstellung erfolgte nach einer Vorschrift von Schellenberger et al. (Coll. Czech. Chem. Commun. 53 (1988) 2884): Verschiedene Benzyloxycarbonylaminosäuren werden mit äquimolaren Mengen 2-Dimethylaminoethanol in Ethylacetat unter Zugabe von Dicyclohexylcarbodiimid verestert. Nach 30 min. wird Dicyclohexylharnstoff abfiltriert und das Produkt mit Ether ausgefällt. Dieses Produkt wird anschließend ohne weitere Charakterisierung in Methyljodid gelöst. Nach 2 Tagen wird der Überschuß an Methyljodid unter Vakuum abgezogen. Die resultierenden Aminosäurecholinesterjodide werden schließlich aus Ethanol umkristallisiert.

Alternativ kann als anionischer Rest eine Sulfonsäuregruppe eingeführt werden. Andere ladungstragende Gruppen stehen dem Fachmann ohne weiteres zur Verfügung.

### Beispiel 17: Enzymatische Dipeptidsynthese mit Benzyloxycarbonylphenylalanincholinester

Benzyloxycarbonylphenylalanincholinester wurde mit äquimolarer Menge Argininamid als Nucleophil und α-Chymotrypsin als Hydrolase umgesetzt. Das Produkt, Benzyloxycarbonylphenylalaninargininamid wurde mittels HPLC identifiziert.

### Beispiel 18: Dipeptide mit C-terminaler anionischer löslicher Schutzgruppe (Sulfonsäureester):

Mit dem Enzym α-CT und Mal-Tyr-OEt als Elektrophil sowie Alaninethylsulfonsäureester (Ala-O(CH₂)₂-OSO₃⁻) als Nukleophil wurde in einem 100-ml Becherglas Mal-Tyr-Ala-O(CH₂)₂-OSO₃⁻ hergestellt. Nach 40 Minuten Reaktionszeit trat keinerlei Änderung im HPLC mehr auf. Das Enzym wurde über ein Ultrafilter (10.000 MWCO) abgetrennt und das Produkt neben den Edukten Mal-Tyr-OEt und Ala-O(CH₂)₂-OSO₃⁻ in etwa gleichen Teilen über LC-MS nachgewiesen.

| | |
|---|---|
| Elektrophil-Nukleophil-Verhältnis | 1:1 |
| Substratkonzentration | je 0,29 M |
| Substrat-Enzym-Verhältnis | 5,6·10⁵ |
| pH-Wert | 9,5 |
| Temperatur | 25°C |

### Beispiel 19: Dipeptide mit C-terminaler anionischer löslicher Schutzgruppe (Sulfonsäureester) im Elektrophil: N-Ac-Tyr-Arg-OEt

Mit dem Enzym α-CT und N-Ac-Tyr-O(CH₂)₂-OSO₃⁻ als Elektrophil sowie Argininethylester (Arg-OEt) als Nukleophil wurde in einem 100-ml Becherglas N-Ac-Tyr-Arg-OEt hergestellt. Nach kurzer Zeit fiel das Produkt als weißer Niederschlag aus und wurde abfiltriert, mit Wasser gewaschen und bei 50°C im Vakuum getrocknet.

| | |
|---|---|
| Elektrophil-Nukleophil-Vernältnis | 1:1 |
| Substratkonzentration | je 0,38 M |
| Substrat-Enzym-Verhältnis | 9,6·10⁵ |
| pH-Wert | 9,5 |
| Temperatur | 25°C |
| Reaktionszeit | 20 Minuten |
| Isolierausbeute | 63% |

Gemäß allg. Definition betrifft die Erfindung ein Verfahren zur Herstellung geschützter bzw. ungeschützter Di- oder Oligopeptide durch enzymatische Umsetzung einer Verbindung der allgemeinen Formel I

X-E-R¹ (I)

als Elektrophil,
in der E unter Einbezug der Gruppen X und R¹ für eine N-terminal mit X und C-terminal mit R¹ substituierte Aminosäure bzw. ein solches Di- oder Oligopeptid steht,
mit einer Verbindung der allgemeinen Formel II

H₂N-Q-R² (II)

als Nucleophil, in der Q unter Einbezug von H₂N und R² für eine C-terminal eine R²-Gruppe tragende Aminosäure oder ein solches Di- oder Oligopeptid steht,
in wäßriger Lösung in Gegenwart einer Hydrolase (E.C. 3.4 ...),
das sich dadurch auszeichnet,
daß mindestens eine der Gruppen X, R¹ oder R² oder die beiden Gruppen X und R¹ zusammen eine bei den zur Umsetzung angewandten pH-Werten die Wasserlöslichkeit der zugehörigen Verbindung erhöhende Gruppe ist,
wobei
Ⓐ
   X gegenüber einer Verbindung der allgemeinen Formel V

   E-R¹ (V),

   in der E unter Einbezug von R¹ für eine C-terminal mit R¹ substituierte Aminosäure bzw. ein solches Di- oder Oligopeptid steht, wobei R¹ nicht OH ist, oder
   R¹ gegenüber einer Verbindung der allgemeinen Formel VI

   X-E-OCH₃ (VI),

   in der E unter Einbezug von X und OCH₃ für eine N-terminal mit X und C-terminal mit OCH₃ substituierte Aminosäure bzw. ein solches Di- oder Oligopeptid steht, wobei R¹ nicht OH ist,
   oder
   X und R¹ zusammen gegenüber einer Verbindung der allgemeinen Formel VII

   CH₃-C(O)-E-OCH₃ (VII),

   in der E unter Einbezug von CH₃-C(O)- und -OCH₃ für eine N-terminal mit CH₃-C(O)- und C-terminal mit -OCH₃ substituierte Aminosäure bzw. ein solches Di- oder Oligopeptid steht, wobei X nicht H und R¹ nicht OH ist,
   und/oder wobei
Ⓑ
   R² gegenüber einer Verbindung der allgemeinen Formel VIII

   H₂N-Q-OCH₃ (VIII),

   in der Q unter Einbezug von H₂N- und -OCH₃ für eine C-terminal eine -OCH₃-Gruppe tragende Aminosäure oder ein solches Di- oder Oligopeptid steht, wobei R² nicht OH ist,
   die Wasserlöslichkeit um einen Faktor > 5 erhöht, und daß das Elektrophil in einer Konzentration ≥ 100 mM, eingesetzt wird.

Das im Vorstehenden beschriebene erfindungsgemäße Verfahren erweist sich als hervorragend geeignet zur wirtschaftlichen Herstellung von Di- und Oligopeptiden durch Umsetzung von N-terminal geschützten Aminosäureestern mit Aminosäure- oder Di- bzw. Oligopeptidestern oder -amiden bzw. den entsprechenden freien Säuren.

## Patentansprüche

1. Verfahren zur Herstellung von geschützten oder ungeschützten Di- oder Oligopeptiden durch enzymatische Umsetzung eines Elektrophils (A) und eines Nucleophils (B) in Gegenwart einer Hydrolase (E.C. 3.4. ...) in wäßriger Lösung, bei der das Nucleophil (B)
- eine Aminosäure oder ein Di- oder Oligopeptid
- oder eine Aminosäure, ein Di- oder Oligopeptid ist, welches als C-terminales Ester oder als C-terminales Amid vorliegt,
dadurch gekennzeichnet,
daß als Elektrophil (A) eine Aminosäure, ein Dipeptid oder ein Oligopeptid in einer Konzentration von ≥ 100 mM eingesetzt wird, welche bzw. welches eine die Wasserlöslichkeit um einen Faktor > 5 gegenüber dem ungeschützten Elektrophil (A) erhöhende N-terminale Schutzgruppe und/oder einer entsprechend C-terminalen Ester- oder Amidgruppe aufweist.

2. Verfahren nach Anspruch 1,
dadurch gekennzeichnet,
daß das Elektrophyl (A) unter Umsetzungs-pH eine ladungstragende N-terminale Schutzgruppe aufweist.

3. Verfahren nach Anspruch 1 oder 2,
dadurch gekennzeichnet,
daß ein Nucleophil (B) mit einer die Wasserlöslichkeit erhöhenden C-terminalen Ester- oder Amidgruppe eingesetzt wird.

4. Verfahren nach einem der Ansprüche 1 bis 3,
dadurch gekennzeichnet,
daß man das als Reaktionsprodukt gebildete Di-oder Oligopeptid abtrennt, von Schutzgruppen befreit und isoliert.

5. Verfahren zur Herstellung geschützter bzw. ungeschützter Di- oder Olipeptide nach Anspruch 1, **dadurch gekennzeichnet,** daß eine Verbindung der allgemeinen Formel I
X-E-R¹ (I)
in der E unter Einbezug von und R¹ fûr eine N-terminal mit X und C-terminal mit R¹ substituierte Aminosäure bzw. ein solches Di- oer Oligopeptid steht, wobei
R¹ eine veresternde Alkylgruppe mit 1 - 4 C-Atomen bedeutet, die ggf. eine Arylgruppe als Substituenten aufweist und
X eine bei den zur Umsetzung angewandten pH-Werten die Wasserlöslichkeit gegenüber Verbindungen mit; X = H um einen Faktor > 5 erhöhende Gruppe ist,
als Elektrophil mit einer Verbindung der allgemeinen Formel II
H₂N-Q-R² (II)
als Nucleophil, in der Q unter Einbezug von H₂N und R² für eine C-terminal eine R²-Gruppe tragende Aminosäure oder ein solches Di- oder Oligopeptid steht, wobei
R² ein freies Säureende, eine ggf. eine Arylgruppe als Substituenten aufweisende C₁ - bis C₄-Alkylveresterung oder eine Amidierung durch -NR³R⁴ symbolisiert, deren Reste R³ und R⁴ jeweils unabhängig voneinander Wasserstoff, einen C₁- bis C₄-Alkylrest, einen solchen Arylalkylrest oder einen Arylrest bedeuten,
in wäßriger Lösung in Gegenwart einer Hydrolase umgesetzt wird, wobei das Elektrophil (I) in ≥ 100 mM Konzentration eingesetzt wird und wobei ggf. R¹ und/oder R², abweichend von der vorstehenden Definition derselben, die Funktion der Löslichkeitsvermittlung im wässrigen Milieu an Stelle von oder zusätzlich zu der Gruppe X, insb. durch kationische oder anionische Struktur, übernehmen bzw. mitübernehmen und X dann ggf. eine beliebige N-terminal Schutzgruppe ist und daß ggf. das von der Reaktionsmischung abgetrennte Reak-tionsprodukt von Schutzgruppen befreit wird.

6. Verfahren nach einem der Ansprüche 1 bis 5,
**dadurch gekennzeichnet,**
daß man die Verbindung der allgemeinen Formel I in einer Konzentration von 100 mM - 1000 mM einsetzt.

7. Verfahren nach einem der vorangehenden Ansprüche,
**dadurch gekennzeichnet,**
daß man das Nucleophil der allgemeinen Formel II in einem Molverhältnis zur Verbindung der allgemeinen Formel I von < 1,5 einsetzt.

8. Verfahren nach einem der vorangehenden Ansprüche,
**dadurch gekennzeichnet,**
daß man mit einem Verbindung der Formel I/Hydrolaseverhältnis von ≥ 10⁵ (M/M) bzw. im Falle von Papain als Hydrolase ≥ 10³ arbeitet.

9. Verfahren nach einem der vorangehenden Ansprüche,
**dadurch gekennzeichnet,**
daß man die Umsetzung kontinuierlich mit Verweilzeiten < 20 min durchfürt.

10. Verfahren nach einem der vorangehenden Ansprüche
**dadurch gekennzeichnet,**
daß R² in der allgemeinen Formel II eine ladungstragende Gruppe darstellt.

11. Verfarhen nach einem der vorangehenden Ansprüche,
**dadurch gekennzeichnet,**
daß, wenn E in der allgemeinen formel I für den Rest einer aromatischen Aminosäure, insb. für L-Tyrosin, L-Phenylalanin oder L-Tryptophan steht, α-Chymotrypsin als Hydrolase verwendet wird.

12. Verfahren nach einem der vorangehenden Ansprüche,
**dadurch gekennzeichnet,**
daß X in der allgemeinen Formel I eine Gruppe der allgemeinen Formel III 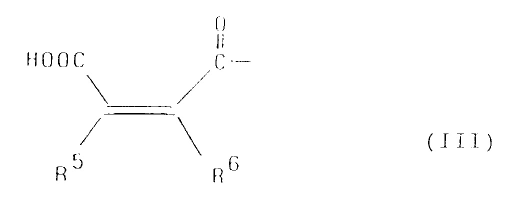 ist, wobei R⁵ und R⁶ jeweils unabhängig voneinander H, einen C₁ - C₄-Alkyl-, Aryl-, Heteroaryl-,

13. N-Betainyl-Aminosäure- oder -Di- bzw. Oligopeptidalkylester der allgemeinen Formel IV 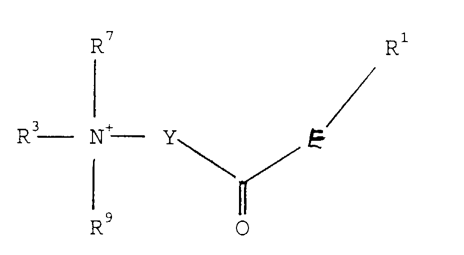 in der E unter Einbezug von X und R¹ für eine N-terminal mit X und C-terminal mit R¹ substituierte Aminosäure bzw. ein solches Di- oder Oligopeptid steht, wobei
R¹ eine veresternde Alkylgruppe mit 1 - 4 C-Atomen bedeutet, die ggf. eine Arylgruppe als Substituenten aufweist und
X eine bei den zur Umsetzung angewandten pH-Werten die Wasserlöslichkeit gegenüber Verbindungen mit X = H um einen Faktor >5 erhöhende Gruppe ist, wobei 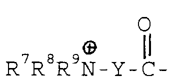 gleich X ist.
Y ein Alkylengruppe mit 1 - 4 C-Atomen, Arylen- oder Aralkylengruppe darstellt und
R⁷, R⁸ und R⁹ jeweils unabhängig voneinander für einen Alkylrest mit 1 - 4 C-Atomen oder einen Arylrest stehen, die ggf. substituiert sein können.
Arylakyl-, Heteroarylalkylrest oder unter Einbeziehung der Doppelbindung zusammen einen ggf. substituierten aromatischen oder alicyclischen Ring bilden, der ggf. auch gesättigt sein kann.

14. Verwendung der Verbindung nach Anspruch 13 zur Herstellung von Peptiden nach einem der in den Ansprüchen 1 bis 11 genannten Verfahren.

15. Verfahren nach Anspruch 1 zur Herstellung geschützter bzw. ungeschützter Di- oder Oligopeptide durch enzymatische Umsetzung einer Verbindung der allgemeinen Formel I
X-E-R¹ (I)
als Elektrophil,
in der E unter Einbezug der Gruppen X und R¹ für eine N-terminal mit X und C-terminal mit R¹ substituierte Aminosäure bzw. ein solches Di- oder Oligopeptid steht,
mit einer Verbindung der allgemeinen Formel II
H₂N-Q-R² (II)
als Nucleophil, in der Q unter Einbezug von H₂N und R² für eine C-terminal eine R²-Gruppe tragende Aminosäure oder ein solches Di- oder Oligopeptid steht,
in wäßriger Lösung in Gegenwart einer Hydrolase (E.C. 3.4 ...),
**dadurch gekennzeichnet,**
daß mindestens eine der Gruppen X, R¹ oder R² oder die beiden Gruppen X und R¹ zusammen eine bei den zur Umsetzung angewandten pH-Werten die Wasserlöslichkeit der zugehörigen Verbindung erhöhende Gruppe ist,
Ⓐ
X gegenüber einer Verbindung der allgemeinen Formel V
E-R¹ (V),
in der E unter Einbezug von R¹ für eine C-terminal mit R¹ substituierte Aminosäure bzw. ein solches Di- oder Oligopeptid steht, wobei R¹ nicht OH ist, oder
R¹ gegenüber einer Verbindung der allgemeinen Formel VI
X-E-OCH₃ (VI),
in der E unter Einbezug von X und OCH₃ für eine N-terminal mit X und C-terminal mit OCH₃ substituierte Aminosäure bzw. ein solches Di- oder Oligopeptid steht, wobei R¹ nicht OH ist,
oder
X und R¹ zusammen gegenüber einer Verbindung der allgemeinen Formel VII
CH₃-C(O)-E-OCH₃ (VII),
in der E unter Einbezug von CH₃-C(O)- und -OCH₃ für eine N-terminal mit CH₃-C(O)- und C-terminal mit -OCH₃ substituierte Aminosäure bzw. ein solches Di- oder Oligopeptide steht, wobei X nicht H und R¹ nicht OH ist,
und/oder wobei
Ⓑ
R² gegenüber einer Verbindung der allgemeinen Formel VIII
H₂N-Q-OCH₃ (VIII),
in der Q unter Einbezug von H₂N- und -OCH₃ für eine C-terminal eine -OCH₃-Gruppe tragende Aminosäure oder ein solches Di- oder Oligopeptid steht, wobei R² nicht OH ist, die Wasserlöslichkeit um einen Faktor > 5 erhöht, und daß das Elektrophil in einer Konzentration ≥ 100 mM, eingesetzt wird.

## Claims

1. Method for the preparation of protected or unprotected dipeptides or oligopeptides by enzymatic reaction of an electrophile (A) and a nucleophile (B) in the presence of a hydrolase (E.C. 3.4. ...) in aqueous solution, in which the nucleophile (B) is
- an amino acid or a dipeptide or oligopeptide
- or an amino acid, a dipeptide or oligopeptide present in the form of a C-terminal ester or a C-terminal amide,
characterised in that
as the electrophile (A) an amino acid, a dipeptide or an oligopeptide is employed, in a concentration of ≥ 100 mM, which has an N-terminal protecting group that increases the solubility in water by a factor > 5 relative to the unprotected electrophile (A), and/or which has a corresponding C-terminal ester or amide group.

2. Method according to claim 1,
characterised in that the electrophile (A) has a charge-carrying N-terminal protecting group below reaction pH.

3. Method according to claim 1 or 2,
characterised in that a nucleophile (B) having a C-terminal ester or amide group that increases the solubility in water is employed.

4. Method according to any of claims 1 to 3,
characterised in that the dipeptide or oligopeptide formed as the reaction product is separated, its protecting groups removed, and isolated.

5. Method for the preparation of protected or unprotected dipeptides or oligopeptides according to claim 1, characterised in that a compound having the general formula I
X-E-R¹ (I)
in which E including X and R¹ stands for an amino acid substituted N-terminally with X and C-terminally with R¹ or for one such dipeptide or oligopeptide, where
R¹ signifies an esterifying alkyl group with 1 to 4 carbon atoms and, if appropriate, having an aryl group as substituent, and
X is a group that at the pH values applied for the reaction increases the solubility in water by a factor > 5 relative to compounds in which X = H,
as the electrophile,
is employed with a compound having the general formula II
H₂N-Q-R² (II)
as the nucleophile, in which Q including H₂N and R² stands for an amino acid carrying an R² group C-terminally or for one such dipeptide or oligopeptide, where
R² denotes a free acid end, a C₁ to C₄ alkyl esterification, having an aryl group as substituent if appropriate, or an amidification by -NR³R⁴, of which the groups R³ and R⁴ in each case independently signify hydrogen, a C₁ to C₄ alkyl group, one such arylalkyl group or an aryl group,
is reacted in aqueous solution in the presence of a hydrolase, the electrophile (I) being employed in a concentration of ≥ 100 mM,
and where, if appropriate, R¹ and/or R², in a variation from the definition thereof given above, assume or jointly assume the function of imparting solubility in the aqueous medium in lieu of or in addition to the group X, more particularly by a cationic or anionic structure, and if appropriate X is then any desired N-terminal protecting group,
and that if appropriate the protecting groups are removed from reaction product after it is separated from the reaction mixture.

6. Method according to any of claims 1 to 5,
characterised in that the compound having the general formula I is employed in a concentration of 100 mM to 1000 mM.

7. Method according any of the preceding claims,
characterised in that the nucleophile having the general formula II is employed in a molar ratio of < 1.5 to the compound having the general formula I of.

8. Method according to any of the preceding claims,
characterised by working with a ratio of ≥ 10⁵(M/M) of formula I compound/hydrolase, or of ≥ 10³ in the event that the hydrolase is papain.

9. Method according to any of the preceding claims,
characterised in that the reaction is carried out continuously with dwell times < 20 minutes.

10. Method according to any of the preceding claims,
characterised in that R² represents a charge-carrying group in the general formula II.

11. Method according to any of the preceding claims,
characterised in that α-chymotrypsin is used as the hydrolase when E in the general formula I stands for the radical of an aromatic amino acid, more particularly for L-tyrosine, L-phenylalanine or L-tryptophan.

12. Method according to any of the preceding claims,
characterised in that X in the general formula I is a group having the general formula III 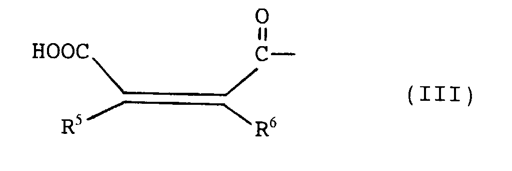 wherein R⁵ and R⁶ are in each case independently hydrogen, a C₁ - C₄ alkyl group, aryl group, heteroaryl group, arylalkyl group, heteroarylalkyl group or including the double bond together form an aromatic or alicyclic ring, substituted if appropriate, which if appropriate may also be saturated.

13. N-betainyl amino acid or dipeptide or oligopeptide alkyl ester having the general formula IV 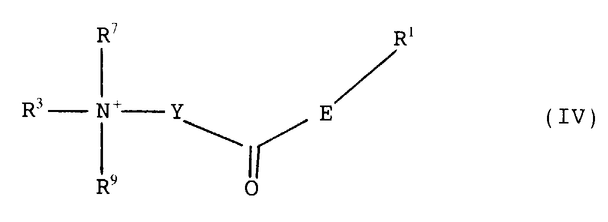 in which E including X and R' stands for an amino acid substituted N-terminally with X and C-terminally with R¹, or one such dipeptide or oligopeptide, where
R¹ signifies an esterifying alkyl group with 1 to 4 carbon atoms, if appropriate having an aryl group as substituent, and
X is a group that at the pH values applied for the reaction increases the solubility in water by a factor > 5 relative to compounds in which X = H,
where 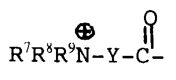 equals X,
Y represents an alkylene group having 1 to 4 carbon atoms, an arylene or aralkylene group, and
R⁷, R⁸ and R⁹ in each case independently stand for an alkyl group having 1 to 4 carbon atoms or an aryl group, these being substituted if appropriate.

14. Use of the compounds according to claim 13 for the preparation of peptides according to any of the methods specified in claims 1 to 11.

15. Method according to claim 1 for the preparation of protected or unprotected dipeptides or oligopeptides by enzymatic reaction of a compound having the general formula I
X-E-R¹ (I)
as the electrophile,
in which E including the groups X and R¹ stands for an amino acid substituted N-terminally with X and C-terminally with R¹, or for one such dipeptide or oligopeptide,
with a compound having the general formula II
H₂N-Q-R² (II)
as the nucleophile, in which Q including H₂N and R² stands for an amino acid carrying an R² group C-terminally or one such dipeptide or oligopeptide,
in aqueous solution in the presence of a hydrolase (E.C. 3.4. ...),
characterised in that at least one of the groups X, R¹ or R², or the two groups X and R¹ together, is a group that at the pH values applied for the reaction increases the solubility in water of the associated compound, where
Ⓐ
X relative to a compound having the general formula V
E-R¹ (V),
in which E including R¹ stands for an amino acid substituted C-terminally with R¹, or for one such dipeptide or oligopeptide, where R¹ is not OH,
or
R¹ relative to a compound having the general formula VI
X-E-OCH₃ (VI),
in which E including X and OCH₃ stands for an amino acid substituted N-terminally with X and C-terminally with OCH₃, or for one such dipeptide or oligopeptide, where R¹ is not OH,
or
X and R¹ together relative to a compound having the general formula VII
CH₃-C(O)-E-OCH₃ (VII),
in which E including CH₃-C(O)- and -OCH₃ stands for
an amino acid substituted N-terminally with CH₃-C(O)- and C-terminally with -OCH₃, or for one such dipeptide or oligopeptide, where X is not H and R¹ is not OH, and/or where
Ⓑ
R² relative to a compound having the general formula VIII
H₂N-Q-OCH₃ (VIII),
in which Q including H₂N- and -OCH₃ stands for an amino acid carrying an -OCH₃ group C-terminally, or for one such dipeptide or oligopeptide, where R² is not OH,
increases the solubility in water by a factor > 5, and that the electrophile is employed in a concentration ≥ 100 mM.

## Revendications

1. Procédé de préparation de dipeptides et d'oligopeptides, protégés ou non protégés, par réaction enzymatique d'un électrophile (A) et d'un nucléophile (B) en présence d'une hydrolase (E.C. 3.4. ...) en solution aqueuse dans laquelle le nucléophile (B) est
- un amino-acide ou un di- ou oligopeptide,
- ou un amino-acide, un di- ou un oligopeptide qui est présent sous la forme d'un ester C-terminal ou d'un ester C-terminal,
caractérisé en ce qu'on utilise, comme électrophile (A), un amino-acide, un dipeptide ou un oligopeptide à une concentration ≥ 100 mM, qui présente un groupe protecteur N-terminal et/ou un groupe ester ou amide C-terminal correspondant, qui augmente la solubilité dans l'eau d'un facteur > 5 par rapport à l'électrophile (A) non protégé.

2. Procédé selon la revendication 1, caractérisé en ce que l'électrophile (A) présente un groupe protecteur N-terminal portant une charge au pH de réaction.

3. Procédé selon la revendication 1 ou 2, caractérisé en ce qu'on utilise un nucléophile (B) ayant un groupe ester ou amide C-terminal qui augmente la solubilité dans l'eau.

4. Procédé selon l'une des revendications 1 à 3, caractérisé en ce qu'on sépare le dipeptide ou l'oligopeptide formé comme produit de réaction, on le débarrasse des groupes protecteurs et on l'isole.

5. Procédé de préparation de dipeptides ou d'oligopeptides protégés ou non protégés selon la revendication 1, caractérisé en ce qu'on fait réagir un composé répondant à la formule générale I
X-E-R¹ (I)
où E, conjointement avec X et R¹, est un amino-acide ou un tel di- ou oligopeptide substitué par X en position N-terminale et par R¹ en position C-terminale, où
R¹ signifie un groupe alkyle estérifiant ayant de 1 à 4 atomes de carbone, qui présente éventuellement un groupe aryle comme substituant, et
X est un groupe qui, aux valeurs de pH utilisées pour la réaction, augmente la solubilité dans l'eau d'un facteur > 5 par rapport à des composés dans lesquels X = H,
en tant qu'électrophile,
avec un composé répondant à la formule générale II
H₂N-Q-R² (II)
en tant que nucléophile, où Q, conjointement avec H₂N et R², représente un amino-acide portant un groupe R² en position C-terminale ou un tel di- ou oligopeptide, où
R² symbolise une terminaison libre d'acide, une estérification par un groupe alkyle en C₁ à C₄ présentant éventuellement un groupe aryle comme substituant ou une amidation par -NR³R⁴ dont les restes R³ et R⁴ signifient chacun, indépendamment l'un de l'autre, l'hydrogène, un reste alkyle en C₁ à C₄, un tel reste arylalkyle ou un reste aryle,
en solution aqueuse, en présence d'une hydrolase, en utilisant l'éleccrophile (I) à une concentration ≥ 100 mM et éventuellement, R¹ et/ou R², ayant une définition différente de la précédente, remplissent la fonction de solubilisation dans le milieu aqueux à la place ou en plus du groupe X, en particulier au moyen d'une structure cationique ou anionique, ou alors ils remplissent cette fonction conjointement avec X, et X est alors éventuellement un groupe protecteur N-terminal quelconque,
et en ce qu'on débarrasse le produit de réaction, séparé du mélange de réaction, des groupes protecteurs.

6. Procédé selon l'une des revendications 1 à 5, caractérisé en ce qu'on utilise le composé de formule I à une concentration de 100 mM à 1 000 mM.

7. Procédé selon l'une des revendications précédentes, caractérisé en ce qu'on utilise le nucléophile répondant à la formule générale II selon un rapport molaire de celui-ci au composé de formule générale I < 1,5.

8. Procédé selon l'une des revendications précédentes, caractérisé en ce qu'on travaille avec un rapport du composé de formule I à l'hydrolase ≥ 10⁵ (M/M) ou ≥ 10³ dans le cas de la papaïne comme hydrolase.

9. Procédé selon l'une des revendications précédentes, caractérisé en ce qu'on effectue la réaction en continu avec des temps de séjour < 20 minutes.

10. Procédé selon l'une des revendications précédentes, caractérisé en ce que R² dans la formule générale II représente un groupe portant une charge.

11. Procédé selon l'une des revendications précédentes, caractérisé en ce qu'on utilise de l'α-chymotrypsine comme hydrolase quand E dans la formule générale I représente le reste d'un amino-acide aromatique, en particulier la L-tyrosine, la L-phénylalanine ou le L-tryptophane.

12. Procédé selon l'une des revendications précédentes, caractérisé en ce que X dans la formule générale I est un groupe répondant à la formule générale III 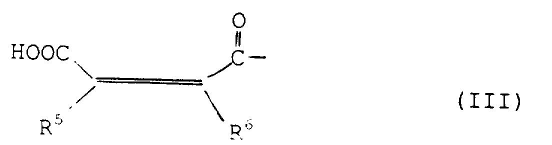 où R⁵ et R⁶ représentent chacun, indépendamment l'un de l'autre H, un reste alkyle en C₁-C₄, aryle, hétéroaryle, arylalkyle, hétéroarylalkyle ou, conjointement avec la liaison double, forment un noyau aromatique ou alicyclique éventuellement substitué et qui peut éventuellement aussi être saturé.

13. Ester d'alkyle et de N-bétaïnyl-amino-acide ou de -di- ou -oligopeptide répondant à la formule générale IV 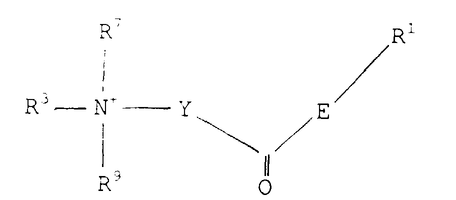 où E, conjointement avec X et R¹, représente un amino-acide substitué par X en position N-terminale et par R¹ en position C-terminale, ou un tel di- ou oligopeptide,
R¹ signifie un groupe alkyle estérifiant ayant de 1 à 4 atomes de carbone, qui peut éventuellement présenter un groupe aryle comme substituant, et
X est un groupe qui, aux valeurs de pH utilisées pour la réaction, augmente la solubilité dans l'eau d'un facteur > 5 par rapport à des composés dans lesquels X = H, où 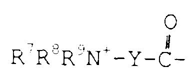 est égal à X,
Y représente un groupe alkylène ayant de 1 à 4 atomes de carbone, un groupe arylène ou un groupe aralkylène, et
R⁷, R⁸ et R⁹ représentent chacun, indépendamment les uns des autres, un reste alkyle ayant de 1 à 4 atomes de carbone ou un reste aryle, qui peuvent éventuellement être substitués.

14. Utilisation des composés selon la revendication 3 pour la preparation de peptides selon l'un des procédés cites sans les revendications 1 à 11.

15. Procédé selon la revendication 1 pour la préparation de dipeptides ou d'oligopeptides, protégés ou non protégés, par réaction enzymatique d'un composé répondant à la formule générale I
X-E-R¹ (I)
comme électrophile,
où E, conjointement avec les groupes X et R¹, représente un amino-acide substitué par X en position N-terminale et par R¹ en position C-terminale, ou un tel di- ou oligopeptide, avec un composé répondant à la formule générale II
H₂N-Q-R² (II)
comme nucléophile, où Q, conjointement avec H₂N et R², représente un amino-acide portant un groupe R² en position C-terminale ou un tel di- ou oligopeptide, en solution aqueuse, en présence d'une hydrolase (E.C. 3.4. ...), caractérisé en ce qu'au moins un des groupes X, R¹ ou R² ou les deux groupes X et R¹ ensemble représentent un groupe qui augmente la solubilité dans l'eau du composé associé aux valeurs de pH utilisées pour la réaction,
Ⓐ
X augmente la solubilité dans l'eau d'un facteur > 5 par rapport à un composé répondant à la formule générale V
E-R¹ (V),
où E, conjointement avec R¹, représente un amino-acide substitué par R¹ en position C-terminale, ou un tel di- ou oligopeptide, R¹ n'étant pas OH,
ou
R¹ augmente la solubilité dans l'eau d'un facteur > 5 par rapport à un composé répondant à la formule générale VI
X-E-OCH₃ (VI),
où E, conjointement avec X et OCH₃, représente un amino-acide substitué par X en position N-terminale et par OCH₃ en position C-terminale ou un tel di- ou oligopeptide, R¹ n'étant pas OH,
ou
X et R¹ ensemble augmentent la solubilité dans l'eau d'un facteur > 5 par rapport à un composé répondant à la formule générale VII
CH₃-C(O)-E-OCH₃ (VII),
où E, conjointement avec CH₃-C(O)- et -OCH₃, représente un amino-acide substitué par CH₃-C(O) en position N-terminale et par -OCH₃ en position C-terminale, ou un tel di- ou oligopeptide, n'étant pas H, et R¹ n'étant pas OH, et/ou
Ⓑ
R² augmente la solubilité dans l'eau par rapport à un composé répondant à la formule générale VIII
H₂N-Q-OCH₃ (VIII)
où Q, conjointement avec H₂N- et -OCH₃, représente un amino-acide portant un groupe -OCH₃ en position C-terminale, ou un tel di- ou oligopeptide, R² n'étant pas OH, et en ce qu'on utilise l'électrophile à une concentration ≥ 100 mM.
